Europäisches Patentamt

European Patent Office

Office européen des brevets

(11) Veröffentlichungsnummer : **0 066 857**
**B1**

(12) **EUROPÄISCHE PATENTSCHRIFT**

(45) Veröffentlichungstag der Patentschrift :
09.09.87

(21) Anmeldenummer : 82104893.1

(22) Anmeldetag : 03.06.82

(51) Int. Cl.⁴ : **C 12 N 15/00, C 12 N 9/40,**
C 12 N 1/20, C 13 D 3/00//
C12R1/19

(54) Verfahren zur Herstellung eines Mikroorganismus, welcher alpha-Galactosidase, aber keine Invertase bildet, so erhaltener Mikroorganismus and seine Verwendung.

(30) Priorität : 04.06.81 DE 3122216

(43) Veröffentlichungstag der Anmeldung :
15.12.82 Patentblatt 82/50

(45) Bekanntmachung des Hinweises auf die Patenterteilung : 09.09.87 Patentblatt 87/37

(84) Benannte Vertragsstaaten :
AT BE CH DE FR GB IT LI LU NL SE

(56) Entgegenhaltungen :
US-A- 3 957 578
HOPPE - SEYLER'S ZEITSCHRIFT FUER PHYSIOLOGISCHE CHEMIE, Band 359, Nr. 9, 1978, Seiten 1118-1119, (DE)., R. Mattes et al.: "The translocation of the raffinose operon occurs by IS1-mediated, site-specific recombination"
HOPPE - SEYLER'S ZEITSCHRIFT FUER PHYSIOLOGISCHE CHEMIE, Band 359, Nr. 9, 1978, Seite 1052, (DE)., J. Altenbuchner et al.: "Physical mapping of the raffiose insertion of bacteriophage P1 by restriction endonuclease analysis and electron microscopy"
HOPPE - SEYLER'S ZEITSCHRIFT FUER PHYSIOLOGISCHE CHEMIE, Band 360, Nr. 8, 1979, Seite 1041, (DE)., R. Schmitt et al.: "Function and genetics of raffinose plasmids in escherichia - coli"
Journal of Bacteriology, Aug. 1968, p. 462-471, vol. 96, no. 2, Pibs, Nov. 1978 p. 259-261
Phil. Trans. R. Soc. Fond. B 300, p. 241-248, 1983
Gene und Klone, p. 257-258, 1984
Die Akte enthält technische Angaben, die nach dem Eingang der Anmeldung eingereicht wurden und die nicht in dieser Patentschrift enthalten sind.

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Mattes, Ralf, Dr.
Eyacher Strasse 37
D-8125 Oberhausen (DE)
Erfinder : Beaucamp, Klaus, Dr.
von-Kühlmann-Strasse 15
D-8132 Tutzing (DE)

(74) Vertreter : Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel
Postfach 860820
D-8000 München 86 (DE)

## Beschreibung

Die Erfindung betrifft ein Verfahren zur Herstellung eines Mikroorganismus, welcher α-Galactosidase, die Cofaktoren nicht benötigt, produziert, aber keine Invertase bildet, die Verwendung eines derartigen Mikroorganismus zur Gewinnung von α-Galactosidase und neue Mikroorganismen.

Extrakte aus Zuckerrüben enthalten neben dem hauptsächlich vorkommenden Zuckeranteil Saccharose geringe Mengen Raffinose, ein Tri-saccharid, das aus den Monosacchariden Galactose/Glucose/Fructose besteht. Diese Raffinose hat die unangenehme Eigenschaft, daß sie sich in den Kristallisationsüberständen anreichert und bei Überschreiten von ca. 1 % an der Gesamt-Zuckermasse deren Kristallisation erschwert bzw. in gewissen Verfahren verhindert. Alleine in einer Zuckerfabrik fallen pro Tag während einer ca. 3 monatigen Kampagne 5 bis 10 t Raffinose im Kristallisationsüberstand an. Alleine in der BRD dürfte die Raffinose-Menge pro Tag in der Dimension von mehr als 200 t liegen.

Die Überführung dieser Raffinose in Saccharose ist ein großer wirtschaftlicher Faktor, der bis jetzt nicht oder nur unzureichend bearbeitet werden konnte, da entsprechende Technologien in der gewünschten Präzision nicht zur Verfügung standen. Unter Präzision ist hier zu verstehen eine selektive Abspaltung der Galactose aus dem Raffinose-Molekül unter Verbleib einer vom verwendeten Agens nicht weiterspaltbaren oder abbaubaren Saccharose. Das ist bisher weder chemisch noch enzymatisch möglich. Bekannt sind enzymatische Methoden, bei denen man sich eines natürlich vorkommenden Enzyms, der α-Galactosidase bedient. Dieses Enzym spaltet per Definition die Galactose quantitativ vom Raffinose-Molekül ab. Alle bekannten α-Galactosidase-Präparate sind jedoch bisher grundsätzlich mit einem Enzym verunreinigt, der Invertase, die Saccharose in Glucose und Fructose spaltet. In Anbetracht der hohen Konzentration an Saccharose im Verhältnis zur anwesenden Raffinose (ca. 60 bis 80 : 1) ist auch eine geringfügige Verunreinigung der α-Galactosidase durch Invertase sehr störend, weil erhebliche Anteile der Saccharose weitergespalten werden und damit nur eine störende Verunreinigung gegen eine andere ausgetauscht wird. Entsprechende Verfahren konnten sich daher in der Praxis kaum einführen.

Hier ist anzumerken, daß es unter den bekannten, mit Invertase verunreinigten α-Galactosidasen, die bisher technologisch eingesetzt wurden, vor allem Pilz-Enzyme sind, die wegen ihrer Zugänglichkeit in der Zucker-Technologie Verwendung gefunden haben. Diese haben neben der genannten Verunreinigung noch den Nachteil, daß sie als Pilz-Enzyme ein Aktivitätsmaximum im deutlich sauren pH-Bereich haben, was bei einer großvolumigen Technologie (pro Fabrik mehrere 100 cbm/Stunde) erhebliche Probleme beim Ansäuern und Reneutralisieren aufwirft.

Eine einzige α-Galactosidase ist bekannt, die nach der Aufreinigung Invertase-frei sein soll. Diese α-Galactosidase benötigt jedoch als Kofactoren Mangan-Ionen und NAD, ist außerdem wenig stabil sowie nur in geringer Konzentration in der Biomasse vorhanden. Daher wurde diese α-Galactosidase im Verfahren unter den Bedingungen einer kontinuierlichen Zuckerfabrikation nicht in Betracht gezogen.

Der Erfindung liegt daher die Aufgabe zugrunde, eine α-Galactosidase bereitzustellen, die natürlicherweise absolut frei ist von Invertase. Gleichzeitig soll das Enzym noch folgende Eigenschaften haben :

Es soll sehr nahe beim Neutralpunkt seine höchste Aktivität entfalten, es sollte eine gewisse Temperaturstabilität zeigen und in relativ konzentrierten Zucker-Lösungen bzw. in Konzentraten von Pflanzenextrakten (Zuckerrüben) seine Aktivität über längere Zeit behalten, es soll keine Cofaktoren benötigen.

Zur Lösung dieser Aufgabe geht die Erfindung davon aus, daß es durch die Anwendung der DNA-Neukombination möglich sein könnte, einerseits das mit der α-Galactosidase üblicherweise vergesellschaftete Enzym Invertase durch Deletion des in Invertase kodierenden Genstückes irreversibel zu entfernen, und das für die Kodierung der α-Galactosidase verantwortliche Genstück in einem Vektor zu amplifizieren und als Ergebnis eines hohen Gen-Dosiseffekts und der Veränderung der Regulation das gewünschte Enzym frei von Invertase in höchstmöglicher Konzentration zu erhalten. Idealerweise sollte der bakterielle Rohextrakt selbst oder in einer gleich wie immobilisierten Form einsetzbar sein. Voraussetzung hierfür wäre die Auffindung eines geeigneten Verfahrens, eines geeigneten Operons und geeigneter Restriktionsendonukleasen.

Gelöst wird diese Aufgabe erfindungsgemäß durch ein Verfahren zur Herstellung eines Mikroorganismus, welcher alpha-Galactosidase die Cofaktoren nicht benötigen, aber keine Invertase bildet, welches dadurch gekennzeichnet ist, daß man sowohl eine ein alpha-Galactosidase-Gen enthaltende DNA als auch einen für die zu verwendenden transformierbaren Zellen geeigneten Vektor, der Antibiotikaresistenzgene enthält, mit Restriktionsendonuclease Sal I (EC 3. 1. 23. 37) vollständig spaltet, aus den Fragmenten der das alpha-Galactosidase-Gen enthaltenden DNA ein Bruchstück mit etwa vier Megadalton relativen Molekulargewichts gewinnt, mit der Lösung des mit Sal I gespaltenen Vektors mischt und in Gegenwart von DNA-Ligase rekombiniert unter Bildung einer rekombinanten DNA, die erhaltene rekombinante DNA mit transformierbaren Zellen inkubiert, die transformierten Zellen auf einem Raffinose als einzige C-Quelle enthaltenden Nährboden bebrütet, die gebildeten antibiotikaresistenten Kolonien isoliert und lysiert, aus dem Lysat die Plasmid-DNA isoliert, diese mit Restriktionsendonuclease Hind III (EC 3. 1. 23. 21) oder Eco R1 (EC 3. 1. 23. 3) spaltet, die erhaltene Lösung verdünnt und mit DNA-Ligase behandelt, die erhaltenen renaturierten Plasmide erneut in transformierbare Zellen einschleust, die transformierten Zellen wieder auf einem Raffinose als C-Quelle sowie Antibioticum enthaltenden Nährboden bebrütet und die gebildeten, Raffinose nicht verwertenden Kolonien isoliert.

Eine das $\alpha$-Galactosidase-Gen enthaltende DNA wird vorzugsweise aus E. coli BMTU 2743, DSM 2092 durch Züchtung desselben in einem geeigneten Nährmedium bei erhöhter Temperatur, vorzugsweise 37 bis 42 C, Fällung mit Polyäthylenglycol und Reinigung z. B. durch Dichtegradienten und Phenolextraktionen gewonnen.

Die Spaltung sowohl der das $\alpha$-Galactosidase-Gen enthaltenden DNA als auch der Vektor-DNA erfolgt in an sich bekannter Weise durch Einwirkung von Restriktionsendonuklease Sal I, zweckmäßig bei einer Temperatur zwischen 35 und 37 C, bis die DNA-Moleküle vollständig gespalten sind. Anschließend wird die Endonuklease, zweckmäßig durch Erhitzen, denaturiert. Durch Gelektrophorese werden die erhaltenen Bruchstücke der das $\alpha$-Galactosidase-Gen enthaltenden DNA fraktioniert und das Fragment mit dem relativen Molekulargewicht von etwa 4 Megadalton isoliert.

Die Rekombination dieses Bruchstückes mit dem gespaltenen Vektor erfolgt durch einfaches Vermischen der Lösung und Zusatz einer geeigneten DNA-Ligase. Bevorzugt wird DNA-Ligase des T4-Phagen (EC 6. 5. 1. 1) verwendet, jedoch können auch DNA-Ligasen anderer Herkunft verwendet werden.

Man erhält so die rekombinante DNA, die man in der üblichen Weise mit transformierbaren Zellen (sog. kompetenten Stämmen welche die Fähigkeit zur Aufnahme von DNA aufweisen) mischt, wobei die Transformationsreaktion abläuft.

Als transformierbare Zellen können im Rahmen der Erfindung die hierfür bekannten Zellen verwendet werden. Beispiele für geeignete transformierbare Zellen sind E. coli, Klebsiella pneumoniae, Pseudomonas aeruginosa, Pseudomonas putida, Saccharomyces cerevisiae und Bacillus subtilis. Besonders bevorzugt wird E. coli beispielsweise der Stamm BMTU 2744 DSM 2093. Dies gilt besonders für den ersten Transformationsschritt im Rahmen des erfindungsgemäßen Verfahrens. Für den zweiten Transformationsschritt beim erfindungsgemäßen Verfahren erfolgt die Wahl der transformierbaren Zellen unter Berücksichtigung der sonstigen Eigenschaften dieser Zellen, wie Verwertbarkeit der Zellbestandteile nach Gewinnung der alpha-Galactosidase, Wachstumseigenschaften und Verfügbarkeit. Besonders bevorzugt werden für die zweite Transformation wieder E. coli und Pseudomonas putida.

Die Auswahl des Vektors hängt ab von der Auswahl der transformierbaren Zellen. Bei der Verwendung von E. coli Stämmen als transformierbare Zellen wird vorzugsweise als Vektor das Plasmid pBR 322 (DSM 2089) verwendet, welches im Handel erhältlich ist (Nucl. Acid. Res. (1978) 5, 2721-2728).

Für Saccharomyces-Zellen eignet sich als Vektor beispielsweise das Plasmid pFL1, beschrieben bei Mercereau-Puijalon et al. Gene, Band 11, 163 bis 167 (1980). Für Bacillus subtilis-Zellen eignet sich speziell als Vektor das Plasmid pHV23, beschrieben von B. Michel et al in Gene, Band 12, 147 bis 154 (1980). Für alle anderen oben aufgeführten Mikroorganismen sowie für E. coli eignet sich auch pRSF 1010. Analoges gilt für pKT 230 (beide beschrieben in « Microbial Degradation of Xenobiotics and Recalcitrant Coumpounds » Eds. Hütter und Leisinger, Acad. Press, (1981) London).

Unter den in Betracht kommenden transformierbaren Zellen werden solche bevorzugt, die selbst keine Plasmide enthalten, beispielsweise der oben genannten E. coli BMTU 2744 DSM 2093.

Erfindungswesentlich ist die Verwendung einer das $\alpha$-Galactosidase-Gen enthaltenden DNA sowie die Spaltung zuerst mit Sal I und die Spaltung der nach der ersten Transformation erhaltenen Plasmid-DNA mit Hind III oder Eco R I, da diese Restriktionsendonukleasen eine Spaltung an der für die Erfindung kritischen Stelle der DNA bewirken. Für die Rekombination des 4 Megadalton-Spaltstückes mit dem gespaltenen Vektor kann prinzipiell jede geeignete DNA-Ligase verwendet werden. Bevorzugt wird die DNA-Ligase des T4-Phagen. Die Religierung erfolgt durch einfache Vermischung der jeweiligen Lösungen in Gegenwart von ATP, einer Sulfhydryl-Verbindung und Magnesiumionen.

Um nach der Religierung diejenigen Zellen aufzufinden, welche den das $\alpha$-Galactosidase-Gen enthaltenden Vektor enthalten, erfolgt die Züchtung auf einem Minimalmedium, welches als einzige Kohlenstoffquelle Raffinose sowie die notwendigen Salze enthält. Aufgrund der Verwendung eines Antibiotika-resistenzgene enthaltenden Vektors wird dem Medium vorzugsweise zusätzlich noch ein Antibiotikum beigefügt, gegen welches die Resistenzgene im Vektor enthalten sind. Beispielsweise enthält das Plasmid PBR 322 Resistenzgene gegen Ampicillin und Tetrazyclin, so daß in diesem Falle vorzugsweise eines dieser Antibiotika verwendet wird, welches das Wachstum solcher Zellen unterdrückt, die dieses Gen nach der Transformation nicht enthalten. Unter Minimalmedium wird hier ein Medium verstanden, welches außer den notwendigen Salzen nur Raffinose enthält.

Die Spaltung der hierbei erhaltenen Hybrid-Plasmid DNA mit Hind III oder Eco R I bewirkt die Deletion der für die Expression von Raffinose-Permease und Invertase kodierenden Gensequenzen. Bei Renaturierung durch Einwirkung von DNA- Ligase erhält man daher Vektoren, welche diese im vorgesehenen Prozeß unerwünschten Gene nicht mehr enthalten und für die Transformation der zweiten Stufe verwendet werden, für welche im übrigen die oben zur Transformation der ersten Stufe gemachten Angaben in gleicher Weise gelten.

Die in der zweiten Transformationsstufe erhaltenen Zellen werden selektioniert, indem sie auf einem Komplett-Nährboden mit pH-Indikator gezüchtet werden, welcher Raffinose als C-Quelle und das entsprechende Antibiotikum enthält. Komplett-Nährboden enthält Pepton und Hefeextrakt zusätzlich zu den Bestandteilen des Minimal-Nährbodens. Diejenigen transformierten Zellen, in denen das Gen für eine Raffinose-Permease und Invertase infolge der Deletion nicht mehr vorhanden sind, können Raffinose nicht mehr in die Zellen transportieren und somit nicht verwerten. Dadurch bleibt der pH des Mediums im neutralen Bereich, was wiederum nicht zu einer Farbänderung führt. Sie lassen sich daher von Zellen, in denen diese Gensequenzen nicht deletiert sind und die durch Aufnahme von Raffinose und anschließende Gärung zur Bildung von Säure führen, aufgrund der unterschiedlichen Färbung des Mediums (pH-Indikator) unterscheiden und abtrennen.

Da die zur direkten Raffinose-Verwendung unter dem Einfluß der Raffinose-Permease nicht mehr befähigten Zellen immer noch α-Galactosidase bilden, welche Raffinose spaltet, bilden sich jedoch auch Kolonien der nicht mehr raffinoseverwertenden Zellen. Besonders bevorzugt erfolgt die Unterscheidung zwischen den Zellen mit und ohne Gen-Sequenzen für Raffinose-Permease und Invertase durch Verwendung von Mac Conkey-Platten (Fa. Difco, enthalten Neutralrot als pH-Indikator), da raffinoseverwertende Kolonien sich auf diesen rot färben, Raffinose nicht verwertende Kolonien hingegen weiß bleiben. Die weißen Kolonien werden isoliert und können dann nach den für den zugrunde liegenden Ausgangsmikroorganismus üblichen Methoden gezüchtet werden.

Hierbei ergab sich, daß die neuen Mikroorganismen auch im technischen Maßstab gezüchtet werden konnten und stabil blieben. Die Mikroorganismen lieferten Enzymaktivitäten, die um mehrere Zehnerpotenzen über den Emzymaktivitäten der Ausgangsstämme lagen.

Das gebildete Enzym war frei von Invertasespuren und konnte bei etwa neutralen pH-werten Raffinose in Sacharosekristallisationsüberständen unter technisch anwendbaren Temperaturen spalten. Das Enzym benötigt keine Cofaktoren wie Mn-Ionen und NAD, es erwies sich als stabil und konnte nach üblichen Methoden, wie z. B. Einwirkung auf bromcyanaktivierte unlösliche Polysaccharide, an Träger fixiert werden. Anstelle des isolierten Enzyms kann auch der Mikroorganismus selbst an geeignete Träger fixiert und in dieser Form technologisch eingesetzt werden. Dabei wir die infolge der Permease-Gen Deletion für Raffinose unpassierbar gewordene Zellmembran durch den Fixierungsvorgang wieder für Raffinose durchgängig. In diesem Fall eignen sich die üblichen Fixierungsmethoden.

Die Isolierung der α-Galactosidase aus den neuen Mikroorganismen kann in der für die Gewinnung von α-Galactosidase üblichen Weise erfolgen. Ein zweckmäßiges Verfahren besteht im Aufschluß der Biomasse nach üblichen Methoden wie Hochdruckdispersion, Ultraschallbehandlung und dergleichen. Zweckmäßig werden die für den Ursprungsmikroorganismus geeigneten Aufschlußmethoden angewendet.

Nach dem Aufschluß wird vorzugsweise eine Polyanionenfällung durchgeführt, bei welcher die α-Galactosidase gewöhnlich im Überstand verbleibt. Bevorzugt als Polianionen werden Polyäthylenimine, insbesondere solche mittleren Molekulargewichts.

Aus dem so erhaltenen Überstand kann das Enzym mit Ammoniumsulfat gefällt werden, wobei vorzugsweise die Fraktion 0,8 bis 1,6 M Ammonsulfat gewonnen wird. Durch Dialyse und Lyophilisierung erhält man in hohen Ausbeuten ein mehr als 90 % reines, zum technischen Einsatz geeignetes α-Galactosidase-Präparat.

Unter Verwendung von Plasmid pBR 322 als Vektor und E. coli BMTU 2744, DSM 2093 als transformierbare Zellen wurde nach dem oben beschriebenen Verfahren der neue Mikroorganismus E. coli BMTU 2742, DSM 2091 erhalten, welcher ebenfalls Gegenstand der Erfindung ist.

Der neue Mikroorganismus ist durch folgende Eigenschaften charakterisiert :

Er enthält das Plasmid pBT 102, ist gegen 50 μg/ml Ampicillin resistent und produziert intracellulär α-Galactosidase die keine Cofaktoren benötigt, konstitutiv, aber keine Invertase. Im übrigen unterscheidet er sich nicht vom E. coli K-12- Stamm und kann bei 37 C im Standard-Medium gezüchtet werden, zweckmäßigerweise unter Belüftung.

Nach dem erfindungsgemäßen Verfahren erhaltene neue Mikroorganismen, welche sich durch einen hohen Gehalt an α-Galactosidase mit den oben erwähnten, besonders vorteilhaften Eigenschaften auszeichnen (bis zu 30 % der gesamten löslichen Proteinmenge kann aus α-Galactosidase bestehen) sind in der Lage, ihre neuen Eigenschaften auf andere Mikroorganismen zu übertragen. Die hierdurch gewonnenen Mikroorganismen sind ebenfalls ein Gegenstand der Erfindung.

Eine Möglichkeit der Übertragung besteht z. B. darin, einen nach dem Verfahren der Erfindung erhaltenen Mikroorganismus mit einem Empfängermikroorganismus gemeinsam zu züchten, z. B. auf Agar zu inkubieren. Verwendet man z. B. für die 2. Transformationsstufe des erfindungsgemäßen Verfahrens einen E. coli-Stamm, der das Plasmid RP 4 enthält, (vgl. J. Bacteriol, 108, 1244-1249 (1971), so bildet sich ein Mikroorganismus, welcher neben RP4 auch den Hybrid-Vektor, wie pBT 103, enthält (also 2 Plasmide aufweist). Dieser Mikroorganismus überträgt durch gemeinsame Züchtung mit Pseudomonasstämmen pBT 103 leicht auf letztere Stämme.

Die für das Verfahren jeweils verwendete transformierbare Form der Mikroorganismen, die sogenannten kompetenten Stämme, werden in der dem Fachmann bekannten Weise erhalten. Zweckmäßig erfolgt die Anzüchtung bis zum Ende der exponentiellen Wachstumsphase. Dann werden die Zellen isoliert und in einer eisgekühlten Calziumchloridlösung suspendiert. In dieser Form sind sie zur Aufnahme von DNA befähigt. Bei Verwendung von E. coli wird als Medium für die Züchtung vorzugsweise sogenannte Standard II-Nährbouillon der Firma Merck, Darmstadt, verwendet.

Durch die Erfindung wird ein Verfahren zur Herstellung von Mikroorganismen geschaffen, welche das Enzym α-Galactosidase in sehr hoher Aktivität bilden, ohne dabei gleichzeitig Invertase zu erzeugen.

4

Auf diese Weise wird eine Quelle für α-Galactosidase geschaffen, die sich weit besser zur Spaltung von Raffinose oder anderen alpha-galactosidisch verknüpften Zuckern, z. B. bei der Zuckerkristallisation aus Rübenzucker eignet als die bisher bekannten α-Galactosidase-Präparate. Hierdurch wird es möglich, großtechnisch das Problem der Raffinoseanreicherung bei der Rüben-Zuckergewinnung wirtschaftlich zu lösen. Der erfindungsgemäße Mikroorganismus oder aus demselben erhaltene ungereinigte oder gereinigte α-Galactosidase-Präparate lassen sich aber auch für andere Zwecke in der Lebensmitteltechnologie einsetzen, in denen eine Spaltung von α-galactosidischen Kohlehydraten erwünscht ist. Ein Beispiel hierfür ist die Entfernung von Stachyose aus Sojamehl. Diese Entfernung ist erforderlich, wenn das Sojamehl für Lebensmittelzwecke verwendbar sein soll.

Die folgenden Beispiele erläutern die Erfindung weiter :

Beispiel 1

Aus dem P1 raf als α-Galactosidase-Gen enthaltende DNA tragenden Stamm Escherichia coli BMTU 2743, DSM 2092 werden neue Stämme mit hoher Produktivität für α-Galactosidase durch die folgenden Verfahrensschritte hergestellt :

(1) Herstellung von P1 raf DNA, welche die genetische Information für das raf-Operon trägt aus einem P1 raf-Lysat.

Der Stamm Escherichia coli BMTU 2743, (thr⁻, leu⁻, thi⁻, lacY, tonA, raf⁺, Plts-Lysogen), der von E. coli K-12, BMTU 2744, DSM 2093 abgeleitet ist, wird für vier Stunden bei 30 °C unter Schütteln in einem Liter Nährbouillon gezüchtet, das 1 % Trypton, 0,5 % Hefeextrakt, 0,2 % Glucose und 0,5 % NaCl enthält und auf einen pH-Wert von 7,8 eingestellt war, und bei Erreichen einer optischen Dichte von 0,5 $OD_{650}$ (gemessen bei 650 nm) bei 40 C, zur Hitzeinduktion des temperatursensitiven Phagenrepressors, zwei Stunden lang weitergeschüttelt. Aus dem Mediumüberstand werden die freigesetzten Phagen durch Fällung mit Polyäthylenglycol gewonnen und über Cäsiumchlorid-dichtegradienten gereinigt. Nach Phenolextraktion und Dialyse gegen Puffer (10 mM Tris. HCl, pH 8,0) werden 0,6 mg gereinigter Phagen-DNA erhalten.

(2) Herstellung der Vektor DNA

Zum Clonieren der Fragmente der P1 raf DNA wird DNA des Plasmids pBR 322, einem Vektor, der sowohl Ampicillin- als auch Tetrazyclin-Resistenzgene als Erkennungsgene (Marker) enthält, verwendet. Man verwendet entweder handelsübliches Plasmid oder es wird in folgender Weise hergestellt : Ein Escherichia coli Stamm, der das Plasmid pBR 322 enthält, wird bei 37 C in 1 L Nährbouillon der gleichen Zusammensetzung wie unter (1) bis zum Ende der exponentiellen Wachstumsphase unter Schütteln angezüchtet. Dann wird nach Zugabe von 1501 µg/ml Chloramphenicol für 15 Stunden bei der gleichen Temperatur weitergeschüttelt. Mit Hilfe dieses Verfahrens wird die Plasmid DNA bevorzugt vervielfältigt und in den Bakterien angereichert. Anschließend werden die Bakterienzellen gewonnen, mit Lysozym und einem nichtionischen Detergens lysiert, und das Lysat bei 48000 g 30 Minuten lang zentrifugiert, um die überstehende Flüssigkeit zu gewinnen. Aus dieser werden dann mit Hilfe zweimaliger Cäsiumchlorid-Äthidiumbromid-Gleichgewichtsdichtegradientenzentrifugation, anschließender Phenolextraktion und Dialyse gegen Puffer (10 mM Tris HCl, pH 8,0) 420 µg der Plasmid-DNA gewonnen.

(3) Insertion des raf-Operons aus P1 raf in den Vektor

Je 10 µg der P1 raf DNA und der Vektor DNA werden bei 37 C 1 Stunde lang mit Restriktionsendonuklease Sal I behandelt, um die DNA-Moleküle komplett zu spalten, und dann jeweils 5 Minuten auf 65 C erhitzt.

Die P1 raf DNA wird danach in einem 0,7 %igen Agarosegel durch Elektrophorese fraktioniert. Das Fragment mit dem relativen Molekulargewicht von vier Megadalton wird ausgeschnitten und nach Phenolextraktion und Dialyse in Puffer (10 mM Tris HCl, pH 8,0) in einer Lösung erhalten.

Die Lösung des so gewonnenen Fragments der P1 raf DNA wird mit der Lösung der gespaltenen pBR 322-Vektor DNA vermischt und unter Zugabe von ATP, Dithioerythrit und Magnesiumchlorid für 24 Std. bei 4 C der Einwirkung von DNA-Ligase des T4-Phagen unterworfen. Die so erhaltene Lösung enthält die rekombinante DNA.

(4) Genetische Transformation von E. coli Bakterien mit rekombinanter DNA, die die genetische Information für α-Galactosidase enthält.

Ein normaler E. coli Stamm, (BMTU 2744), (DSM 2093) wird unter Schütteln in 50 ml Nährbouillon bei 37 C bis zum Ende der exponentiellen Wachstumsphase gezüchtet. Die Zellen werden gewonnen und in einer 50 mM $CaCl_2$ Lösung im Eisbad suspendiert.

Diese Suspension wird mit Lösung der rekombinanten DNA aus Stufe (3) vermischt und für 20

5

0 066 857

Minuten im Eisbad gehalten, dann für 3 Minuten auf 37° erwärmt. Die Zellen werden in Nährbouillon überimpft und 45 Minuten lang bei 37 C geschüttelt, wodurch die Transformationsreaktion vervollständigt wird. Die Zellen werden gewonnen, gewaschen und wieder suspendiert. Ein kleiner Teil der Zellsuspension wird auf einer Agar-Platte ausgestrichen, die pro Liter 10,5 g $K_2HPO_4$, 4,5 g $KH_2PO_4$, 1 g $(NH_4)_2SO_4$, 0,5 g Natriumcitrat $\times$ 2 $H_2O$, 0,1 g $MgSO_4 \times$ 7 $H_2O$, 1 mg Thiamin, 2 g Raffinose, 25 mg Ampicillin und 15 g Agar enthält (pH-Wert auf pH 7,2 eingestellt). Die Platte wird bei 37 C bebrütet. Nach zwei Tagen Bebrütung erscheinen zahlreiche Kolonien auf der Platte. Sämtliche Kolonien werden entnommen, gereinigt und isoliert.

Jede der so erhaltenen Kolonien hat die Fähigkeit erlangt, Raffinose als alleinige C-Quelle zu verwerten und ist gleichzeitig Ampicillin-resistent. Sie hat damit andere Eigenschaften als der Stamm der als Wirtsorganismus verwendet wurde. Das bedeutet, daß nur die Zellen als wachsende Kolonien selektioniert werden, die das pBR 322-Plasmid enthalten, in welches das sal I Fragment aus PI raf mit dem $\alpha$-Galactosidase-Gen inseriert worden ist.

Aus den erhaltenen Kolonien wird jeweils die Plasmid DNA nach dem unter (2) beschriebenen Verfahren isoliert. Die Plasmide aus allen Kolonien zeigen nach Behandlung mit Restriktionsendonuklease Eco R I oder Hind III und Analyse durch Elektrophorese in Agarosegelen zwei Fragmente, die je nach Herkunftskolonie des Hybridplasmides zwei verschiedenen Größenklassen zugeordnet werden können, in der Summe aber gleich sind.

Beide Größenklassen werden etwa im Verhältnis 1 : 1 aufgefunden. Die unterschiedlichen Analysenergebnisse für beide Fragment-Größenklassen sind auf die verschiedene Integrationsrichtung des Sal I Fragmentes aus P1 raf in pBR 322 zurückzuführen.

Für die weiteren Arbeiten wir ein Plasmid einer Kolonie ausgewählt, das nach Behandlung mit Restriktionsenzym Eco R I oder Hind III ein kleineres zweites Fragment ergibt als die Plasmide anderer Kolonien. Dieses Plasmid wird pBT 100 (BMTU 2741) benannt (DSM 2090). Bakterien, die dieses Plasmid tragen, können Raffinose als einzige C-Quelle verwenden und produzieren konstitutiv alle Enzyme des Raf-Operons.

(5) Je 10 μg der DNA des Plasmids pBT 100 aus BMTU 2741 (DSM 2090) werden entweder mit der Restriktionsendonuklease Hind III oder mit der Restriktionsendonuklease Eco R I bei 37 C 1 Stunde lang behandelt, um die DNA- Moleküle zu spalten. Dann wird jeweils 5 Minuten auf 65 C erhitzt.

Die so behandelten Plasmid-DNA Lösungen werden verdünnt und jeweils wie unter (3) beschrieben mit T4- Ligase einer Renaturierungsbehandlung unterworfen.

Anschließend werden wie unter (4) beschrieben kompetenten Zellen des E. coli Stammes jeweils Lösungen der renaturierten DNA aus dem letzten Schritt der Transformationsreaktion zugeführt. Ein kleiner Teil der jeweils so behandelten Zellen wird auf je einer Mac Conkey Raffinose Platte (Difco Laboratories Detroit) mit 25 μg/ml Ampicillin ausgestrichen. Auf der Platte aus der Transformation mit vorher Hind III behandelter Plasmid-DNA werden ca. 20 Kolonien nach Bebrütung bei 37 C über 24 Stunden sichtbar, auf der Platte der Transformation von vorher Eco R I behandelter DNA finden sich unter gleichen Bedingungen ca. 301 Kolonien. Das Verhältnis der roten zu den weißen Kolonien beträgt im ersten Fall 1 : 4, im zweiten Fall 1 : 3.

Die roten Kolonien sind jeweils zum Raffinose-Abbau fähig, enthalten also immer noch das gesamte Raf-Operon, die weißen Zellen sind alle nicht zum Raffinose-Abbau befähigt. Diese produzieren aber immer noch konstitutiv das Enzym $\alpha$-Galactosidase wie der Test im zellfreien Extrakt mit p-Nitrophenyl-$\alpha$-galactosid ($\alpha$-PNPG)als Farbsubstrat zeigt, wenn die Zellen vorher ohne Raffinose-Zusatz in Nährbouillon bei 37 C bis zur Stationärphase angezüchtet sind. Diese Zellen besitzen daher andere Eigenschaften als der Wirtsorganismus oder die Zellen BMTU 2741 (DSM 2090), welche mit pBT 100 transformiert sind.

Das Plasmid, das nach vorhergegangener Hind III Behandlung von pBT 100 in der auf Mac Conkey Platten weißen Kolonien erhalten wird, wird pBT 101 benannt, das nach der vorhergegangenen Eco R I Reaktion erhaltene wird pBT 102 genannt. Der E. coli Stamm mit pBT 102 wird BMTU 2742 (DSM 2091) benannt.

(6) Gewinnung von $\alpha$-Galactosidase aus Stämmen mit pBT 102 (BMTU 2742)

Tabelle 1 zeigt die experimentiellen Ergebnisse der Testung des zellfreien Extraktes auf den Gehalt an $\alpha$-Galactosidase mit $\alpha$-PNPG bei 25 C nach Anzucht des Stammes BMTU 2742, der eines der in Stufe (5) erhaltenen Klone darstellt. Die Zellen werden in 10 ml der Nährbouillon in 100 ml Kolben inokuliert und 15 Std. bei 30 C geschüttelt. Kontrollversuche wurden unter Züchtung des Ausgangsstammes BMTU 2743 unter den gleichen Bedingungen, aber unter Zusatz von 0,2 % Raffinose zum Medium, durchgeführt.

Wie aus der Tabelle 1 ersichtlich ist, zeigten die Extrakte des Stammes BMTU 2742 im Vergleich mit BMTU 2743 einen bemerkenswert erhöhten Gehalt an $\alpha$-Galactosidase.

(Siehe Tabelle Seite 7 f.)

6

**0 066 857**

Tabelle 1

| Stamm Nr. | $\alpha$-Gal U/mg * |
|---|---|
| BMTU 2742 (DSM 2091) | 10 |
| 2743 (DSM 2092) | 0,4 |
| | * mg lösliches Protein im Roh-extrakt |

Unter diesen Testbedingungen findet sich im bekannten Stamm E. coli BMTU 2744 eine Aktivität von < 0,001 u/mg.

Beispiel 2

Spaltung von Raffinose im Kristallisationsablauf einer Zuckerfabrik 45 ml Ablauf, die 802 mg Raffinose enthielten, wurden mit 45 ml Wasser verdünnt. Die Lösung wurde auf einen pH-Wert von 6,5 eingestellt. Diese Lösung wurde in Parallelversuchen sowohl mit einem zellfreien Extrakt als auch mit Toluol-behandelten Zellen des erfindungsgemäßen Stammes E. coli DSM 2091 inkubiert, wobei Extrakt bzw. behandelte Zellen je 57 Einheiten $\alpha$-Galactosidase enthielten.

Das Gemisch wurde für 60, 120 und 180 Minuten bei 40 °C belassen. Zu den genannten Zeiten wurde der Galactose- Gehalt der Lösung mittels eines enzymatischen Tests ermittelt und die Restmenge Raffinose im Ansatz kalkuliert.

Nach den angegebenen Behandlungszeiten wurde eine Restmenge Raffinose von 459, 378 und 315 mg entsprechend einer Herabsetzung des ursprünglichen Raffinosegehaltes auf 57,2, 47,1 und 39,2 % gefunden. Die nachstehende Tabelle 2 faßt die erhaltenen Ergebnisse zusammen.

Tabelle 2

90 ml Kristallisationsablauf-Lösung, enthaltend 802 mg Raffinose, behandelt mit 57 U $\alpha$-Galactosidase bei 40 °C und pH 6,5

| Behandlungszeit (min) | Raffinose-Gehalt (mg) | % vom Ausgangswert |
|---|---|---|
| 0 | 802 | 100,0 |
| 60 | 459 | 57,2 |
| 120 | 378 | 47,1 |
| 180 | 315 | 39,2 |

**Patentansprüche** (für die Vertragsstaaten : BE, CH, DE, FR, GB, IT, LI, LU, NL, SE)

1. Verfahren zur Herstellung eines Mikroorganismus, welcher konstitutiv eine alpha-Galactosidase, die Kofaktoren nicht benötigt, aber keine Invertase bildet, dadurch gekennzeichnet, daß man sowohl eine ein alpha-Galactosidase-Gen enthaltende DNA als auch einen für die zu verwendenden transformierbaren Zellen geeigneten Vektor, der Antibiotikaresistenzgene enthält, mit Restriktionsendonuclease Sal I vollständig spaltet, aus den Fragmenten der das alpha-Galactosidase-Gen enthaltenden DNA das Bruchstück mit etwa vier Megadalton relativen Molekulargewichts gewinnt, mit der Lösung des ebenfalls mit Sal I gespaltenen Vektors mischt und in Gegenwart von DNA-Ligase rekombiniert unter Bildung einer rekombinaten DNA, die erhaltene rekombinante DNA mit transformierbaren E. coli-Zellen inkubiert unter Transformation der rekombinanten DNA in die Zellen, die transformierten Zellen auf einem Raffinose als einzige C-Quelle enthaltenden Nährboden bebrütet, die gebildeten antibiotikaresistenten Kolonien isoliert und lysiert, aus dem Lysat die Plasmid-DNA isoliert, diese mit Restriktionsendonuclease Hind III oder Eco R I spaltet, die erhaltene Lösung verdünnt und mit DNA-Ligase behandelt, die erhaltenen renaturierten Plasmide erneut in transformierbare Zellen, die konstitutiv keine Invertase bilden, einschleust, die transformierten Zellen wieder auf einem Raffinose als C-Quelle sowie Antibioticum enthaltenden Nährboden bebrütet und die gebildeten, Raffinose nicht verwertenden Kolonien isoliert.

2. Verfahren nach Anspruch 1, dadurch gekennzeichnet, daß man als alpha-Galactosidase-Gen das Gen aus E. coli DSM 2092 verwendet.

3. Verfahren nach Anspruch 1 oder 2, dadurch gekennzeichnet, daß man für die zweite Transformation mit renaturierten Plasmiden als transformierbare Zellen E. coli Pseudomonas putida oder Bacillus subtilis verwendet.

4. Verfahren nach einem der Ansprüche 1 bis 3, dadurch gekennzeichnet, daß man als Vektor, der Antibiotikaresistenzgene enthält, Plasmid pBR 322 verwendet.

5. Verwendung eines nach dem Verfahren eines der Ansprüche 1 bis 4 erhaltenen Mikroorganismus zur Gewinnung einer von Invertaseaktivität freien alpha-Galactosidase.

6. Mikroorganismus der Gattung E. coli, Pseudomonas putida oder Bacillus subtilis, welcher eine alpha-Galactosidase, die Cofaktoren nicht benötigt, aber keine Invertase bildet, und nach dem Verfahren der Ansprüche 1 bis 4 erhältlich ist.


**Patentansprüche** (für den Vertragsstaat Österreich)

1. Verfahren zur Herstellung eines Mikroorganismus, welcher konstitutiv eine alpha-Galactosidase, die Kofaktoren nicht benötigt, aber keine Invertase bildet, dadurch gekennzeichnet, daß man sowohl eine ein alpha-Galactosidase-Gen enthaltende DNA als auch einen für die zu verwendenden transformierbaren Zellen geeigneten Vektor, der Antibiotikaresistenzgene enthält, mit Restriktionsendonuclease Sal I vollständig spaltet, aus den Fragmenten der das alpha-Galactosidase-Gen enthaltenden DNA das Bruchstück mit etwa vier Megadalton relativen Molekulargewichts gewinnt, mit der Lösung des ebenfalls mit Sal I gespaltenen Vektors mischt und in Gegenwart von DNA-Ligase rekombiniert unter Bildung einer rekombinaten DNA, die erhaltene rekombinante DNA mit transformierbaren E. coli-Zellen inkubiert unter Transformation der rekombinanten DNA in die Zellen, die transformierten Zellen auf einem Raffinose als einzige C-Quelle enthaltenden Nährboden bebrütet, die gebildeten antibiotikaresistenten Kolonien isoliert und lysiert, aus dem Lysat die Plasmid-DNA isoliert, diese mit Restriktionsendonuclease Hind III oder Eco R I spaltet, die erhaltene Lösung verdünnt und mit DNA-Ligase behandelt, die erhaltenen renaturierten Plasmide erneut in transformierbare Zellen, die konstitutiv keine Invertase bilden, einschleust, die transformierten Zellen wieder auf einem Raffinose als C-Quelle sowie Antibioticum enthaltenden Nährboden bebrütet und die gebildeten, Raffinose nicht verwertenden Kolonien isoliert.

2. Verfahren nach Anspruch 1, dadurch gekennzeichnet, daß man als alpha-Galactosidase-Gen das Gen aus E. coli DSM 2092 verwendet.

3. Verfahren nach Anspruch 1 oder 2 dadurch gekennzeichnet, daß man für die zweite Transformation mit renaturierten Plasmiden als transformierbare Zellen E. coli Pseudomonas putida oder Bacillus subtilis verwendet.

4. Verfahren nach einem der Ansprüche 1 bis 3, dadurch gekennzeichnet, daß man als Vektor, der Antibiotikaresistenzgene enthält, Plasmid pBR 322 verwendet.

5. Verwendung eines nach dem Verfahren eines der Ansprüche 1 bis 4 erhaltenen Mikroorganismus zur Gewinnung einer von Invertaseaktivität freien alpha-Galactosidase.

6. Verwendung eines Mikroorganismus gemäß einem der Ansprüche 1 bis 4 oder eines daraus hergestellten alpha-Galactosidasepräparates für die Lebensmitteltechnologie.


**Claims** (for the Contracting States BE, CH, DE, FR, GB, IT, LI, LU, NL, SE)

1. Process for the production of a micro-organism which constitutively forms an alpha-galactosidase, which does not need cofactors, but not invertase, characterised in that one fully splits not only a DNA containing an alpha-galactosidase gene but also a vector suitable for the transformable cells to be used, which contains antibiotic-resistant genes, with restriction endonuclease Sal I, obtains from the fragments of the DNA containing the alpha-galactosidase gene the fragment of about four megadalton relative molecular weight, mixes with the solution of the vector also split with Sal I and recombines in the presence of DNA ligase with formation of a recombinant DNA, incubates the recombinant DNA obtained with transformable E. coli cells with transformation of the recombinant DNA into the cells, cultures the transformed cells on a nutrient medium containing raffinose as sole C-source, isolates and lyses the antibiotic-resistant colonies formed, isolates from the lysate the plasmid DNA, splits this with restriction endonuclease Hind III or Eco R I, dilutes the solution obtained and treats with DNA ligase, again introduces the renatured plasmids obtained into transformable cells which constitutively do not form invertase, again cultures the transformed cells on a nutrient medium containing raffinose as C-source, as well as antibiotic, and isolates the colonies formed which do not utilise raffinose.

2. Process according to claim 1, characterised in that, as alpha-galactosidase gene, one uses the gene from E. coli DSM 2092.

3. Process according to claim 1 or 2, characterised in that, for the second transformation with renatured plasmids, as transformable cells one uses E. coli, Pseudomonas putida or Bacillus subtilis.

4. Process according to one of claims 1 to 3, characterised in that, as vector which contains antibioticresistant genes, one uses plasmid pBR 322.

5. Use of a micro-organism obtained according to the process of one of claims 1 to 4 for the obtaining of an alpha-galactosidase free of invertase activity.

6. Micro-organism of the species E. coli, Pseudomonas putida or Bacillus subtilis which forms an alphagalactosidase, which does not need cofactors, but not invertase and is obtainable according to the process of claims 1 to 4.

**Claims** (for the Contracting State AT)

1. Process for the production of a micro-organism which constitutively forms an alpha-galactosidase, which does not need cofactors, but not invertase, characterised in that one fully splits not only a DNA containing an alpha-galactosidase gene but also a vector suitable for the transformable cells to be used, which contains antibiotic-resistant genes, with restriction endonuclease Sal I, obtains from the fragments of the DNA containing the alpha-galactosidase gene the fragment of about four megadalton relative molecular weight, mixes with the solution of the vector also split with Sal I and recombines in the presence of DNA ligase with formation of a recombinant DNA, incubates the recombinant DNA obtained with transformable E. coli cells with transformation of the recombinant DNA into the cells, cultures the transformed cells on a nutrient medium containing raffinose as sole C-source, isolates and lyses the antibiotic-resistant colonies formed, isolates from the lysate the plasmid DNA, splits this with restriction endonuclease Hind III or Eco R I, dilutes the solution obtained and treats with DNA ligase, again introduces the renatured plasmids obtained into transformable cells which constitutively do not form invertase, again cultures the transformed cells on a nutrient medium containing raffinose as C-source, as well as antibiotic, and isolates the colonies formed which do not utilise raffinose.

2. Process according to claim 1, characterised in that, as alpha-galactosidase gene, one uses the gene from E. coli DSM 2092.

3. Process according to claim 1 or 2, characterised in that, for the second transformation with renatured plasmids, as transformable cells one uses E. coli, Pseudomonas putida or Bacillus subtilis.

4. Process according to one of claims 1 to 3, characterised in that, as vector which contains antibiotic-resistant genes, one uses plasmid pBR 322.

5. Use of a micro-organism obtained according to the process of one of claims 1 to 4 for the obtaining of an alpha-galactosidase free of invertase activity.

6. Use of a micro-organism according to one of claims 1 to 4 or of an alpha-galactosidase preparation produced therefrom for foodstuff technology.

**Revendications** (pour les Etats Contractants : BE, CH, DE, FR, GB, IT, LI, LU, NL, SE)

1. Procédé pour l'obtention d'un microorganisme lequel constitutivement forme une alpha-galactosidase, qui ne nécessite pas de cofacteurs, mais ne forme pas d'invertase, caractérisé en ce que l'on clive totalement aussi bien un ADN contenant un gène de l'alpha-galactosidase qu'également un vecteur approprié aux cellules transformables utilisées, vecteur qui contient le gène de la résistance aux antibiotiques, au moyen de l'endonucléase de restriction Sal I, à partir des fragments de l'ADN contenant le gène de l'alpha-galactosidase, on récupère le fragment ayant environ quatre mégadaltons de poids moléculaire relatif, on mélange avec la solution du vecteur également clivé par la Sal I et on recombine en présence d'ADN-ligase avec formation d'un ADN recombinant, on incube l'ADN recombinant obtenu avec des cellules de E. Coli transformables avec transformation de l'ADN recombinant dans les cellules, on incube les cellules transformées sur un milieu nutritif contenant du raffinose en tant que source unique de C, on isole et lyse les colonies résistantes aux antibiotiques formées, à partir du lysat on isole l'ADN plasmidique, on clive celui-ci au moyen de l'endonucléase de restriction Hind III ou Eco R I, on dilue la solution obtenue et on la traite avec de l'ADN-ligase, on introduit les plasmides renaturés obtenus de nouveau dans des cellules transformables qui ne forment pas de façon constitutive de l'invertase, on incube de nouveau les cellules transformées sur un milieu nutritif contenant du raffinose comme source de C ainsi qu'un antibiotique et on isole les colonies formées n'utilisant pas le raffinose.

2. Procédé selon la revendication 1, caractérisé en ce que l'on utilise en tant que gène de l'alphagalactosidase le gène provenant de E. Coli DSM 2092.

3. Procédé selon la revendication 1 ou 2, caractérisé en ce que l'on utilise pour la deuxième transformation avec les plasmides renaturés, en tant que cellules transformables E. Coli Pseudomonas putida ou Bacillus subtilis.

4. Procédé selon l'une des revendications 1 à 3, caractérisé en ce que l'on utilise comme vecteur qui contient le gène de la résistance aux antibiotiques, le plasmide PBR 322.

5. Utilisation d'un microorganisme obtenu selon le procédé de l'une des revendications 1 à 4, pour l'obtention d'une alpha-galactosidase ne possédant pas d'activité d'invertase.

6. Microorganisme du genre E. Coli Pseudomonas putida ou Bacillus subtilis qui forme une alphagalactosidase, qui ne nécessite pas de cofacteurs mais ne forme pas d'invertase et qui peut être obtenu selon le procédé de l'une des revendications 1 à 4.

**Revendications** (pour l'Etat Contractant AT)

1. Procédé pour l'obtention d'un microorganisme lequel constitutivement forme une alpha-galactosidase, qui ne nécessite pas de cofacteurs, mais ne forme pas d'invertase, caractérisé en ce que l'on clive totalement aussi bien un ADN contenant un gène de l'alpha-galactosidase qu'également un vecteur approprié aux cellules transformables utilisées, vecteur qui contient le gène de la résistance aux antibiotiques, au moyen de l'endonucléase de restriction Sal I, à partir des fragments de l'ADN contenant le gène de l'alpha-galactosidase, on récupère le fragment ayant environ quatre mégadaltons de poids moléculaire relatif, on mélange avec la solution du vecteur également clivé par la Sal I et on recombine en présence d'ADN-ligase avec formation d'un ADN recombinant, on incube l'ADN recombinant obtenu avec des cellules de E. Coli transformables avec transformation de l'ADN recombinant dans les cellules, on incube les cellules transformées sur un milieu nutritif contenant du raffinose en tant que source unique de C, on isole et lyse les colonies résistantes aux antibiotiques formées, à partir du lysat on isole l'ADN plasmidique, on clive celui-ci au moyen de l'endonucléase de restriction Hind III ou Eco R I, on dilue la solution obtenue et on la traite avec de l'ADN-ligase, on introduit les plasmides renaturés obtenus de nouveau dans des cellules transformables qui ne forment pas de façon constitutive de l'invertase, on incube de nouveau les cellules transformées sur un milieu nutritif contenant du raffinose comme source de C ainsi qu'un antibiotique et on isole les colonies formées n'utilisant pas le raffinose.

2. Procédé selon la revendication 1, caractérisé en ce que l'on utilise en tant que gène de l'alphagalactosidase le gène provenant de E. Coli DSM 2092.

3. Procédé selon la revendication 1 ou 2, caractérisé en ce que l'on utilise pour la deuxième transformation avec les plasmides renaturés, en tant que cellules transformables E. Coli Pseudomonas putida ou Bacillus subtilis.

4. Procédé selon l'une des revendications 1 à 3, caractérisé en ce que l'on utilise comme vecteur qui contient le gène de la résistance aux antibiotiques, le plasmide PBR 322.

5. Utilisation d'un microorganisme obtenu selon le procédé de l'une des revendications 1 à 4, pour l'obtention d'une alpha-galactosidase ne possédant pas d'activité d'invertase.

6. Utilisation d'un microorganisme selon l'une des revendications 1 à 4, ou d'une préparation d'alphagalactosidase obtenue à partir de celui-ci pour les technologies alimentaires.